# EUROPEAN PATENT SPECIFICATION

(11) **EP 2 967 915 B1**
(45) Date of publication and mention of the grant of the patent: **23.11.2016**
(21) Application number: 14711594.3
(22) Date of filing: 14.03.2014
(51) Int. Cl.: A61F 2/46

(54) **IMPLANT TRIALLING**
TESTEN VON IMPLANTATEN
ESSAI D'IMPLANT

(30) Priority: 15.03.2013 GB 201304837
(43) Date of publication of application: 20.01.2016
(73) Proprietor: DePuy (Ireland), County Cork (IE)
(72) Inventor: HUNT, Christopher, Leeds LS11 0EA (GB); PRINCE, Stephanie, Leeds LS11 0EA (GB)
(74) Representative: Alton, Andrew
(86) International application number: PCT/GB2014/050819
(87) International publication number: WO 2014/140639

(56) References cited:
- EP-A1- 1 293 179
- WO-A2-2010/129880
- US-A- 5 888 211
- US-A1- 2002 193 882

## Description

The present invention relates to implant trialling and in particular to trial implants for ball and socket type joints.

Prosthetic implants can be used to replace ball and socket type joints such as shoulder joints and hip joints. Many of these prosthetic implants have the general construction of a stem component, which is received in a long bone (such as the femur or humerus) to which a head component is attached and which provides an articulating surface. The head component can be received in a component having a concave articulating surface, sometimes referred to as a cup, so as to reconstitute the full ball and socket type joint.

During the surgical procedure it is often important accurately to place and position the prosthetic components. Typically cavities are formed in the patient's bones, for example using a broach or reamer, and parts of the patient's bones are resected during the surgical procedure, for example using a saw, in order to prepare the bones for implantation of the prosthetic joint. The preparation of the bones is not perfectly reliable and reproducible and so sometimes the actual positioning of the implants in the patient's prepared bones does not correspond to a planned position. Therefore, as part of the surgical procedure, trial components are often used to help assess intra-operatively the position of the implants and determine whether any changes to the implants or the bone preparation may be needed to bring the actual implant position closer to the planned position.

Although ideally trial components would be used, in some instances surgeons may either elect not to use a trial component or the surgeon may want to do some further trialling from the implanted prosthetic component, for example because the position of the actual prosthetic component may differ from that of the trial component. Hence, the surgeon may want to do some trialling from the actual prosthetic component, either for efficiency of work flow or because the position of the prosthetic component is now fixed and therefore provides a more reliable datum. However, as the prosthetic component is not intended for trialling, use of the prosthetic component can render trialling less reliable.

Hence, it would be beneficial to make trialling for ball and socket type joints more reliable.

Implants are often provided in a variety of different sizes and the most appropriate size for an individual patient may not become clear until intra-operatively, when the bones have been prepared. It can therefore be necessary to provide a wide range of sizes of trial components to cover most scenarios. Further, when it is possible to vary a property of the implant, for example the off set, by combining different parts, then a variety of different implant parts can be needed for each different implant size. This can give rise to a large number of trial parts which need to be provided which can lead to confusion, reduced efficiency of the surgical procedure, increased inventory and increased manufacturing difficulty and costs.

An example of a trial implant for a ball and socket type joint with a stem component and an adjustable articulating component is described in US-5,569,263. The articulating component is attached to a neck extending from a stem component and which has a plurality of grooves along its length. A locking mechanism can engage selectively with a one of the grooves to adjust the position of the articulating component relative to the stem to adjust the joint centre of rotation position.

Another adjustable trial implant is described in DE 102008030260. The trial implant has an articulating component which can detachably connect to a further component in different connecting positions relative to each other for example by using a screw thread mechanism.

WO 2010/129880 describes a prosthesis trial system including a plurality of head members having an outer surface and a cavity configured to mate with an exterior surface of a stem member. The prosthesis trial system further includes a plurality of different sized shell members, each having an inner surface configured to mate with the outer surface of the head members. The each head members includes a plurality of projections each with a plurality of barbs. The projections flex radially outwards to receive the stem and the barbs engage a groove on the stem. Each head member provides a different offset owing to the different positions of their outer surfaces relative to the barbs. The shell members include male formations on the inners surface to mate with grooves in the outer surfaces of the head members and also to prevent some shell members from being fully mated with some of the head members.

US 2002/0193882 describes trial balls for hip joint prostheses which have a cone connection between a prosthesis neck having a cone and fittable hip joint balls, with the hip joint balls and the trial balls each having practically the same radii and each having the same position of their conical counter surfaces relative to the ball center. The trial balls have a separate insert fixed within the trial balls and with a pre-determined spring effect for the contact to the cone. The insert includes multiple flexion springs bearing inner and outer cams and the holding force is determined by the inner cams. The insert can be secured in the trial balls at a different spacing along the polar axis to the ball center.

EP 1293179 describes a prosthetic head including a thimble made from titanium secured in the body of the prosthetic head and which deforms when a head spigot is inserted in the body of the head to act as a resilient damping means between the femur and acetabulum.

Hence, it would be beneficial to make trialling for ball and socket type joints simpler.

According to a first aspect of the invention, there is provided a trialling system as defined in claim 1.

Hence, the surgeon can trial using the prosthetic stem, while some or all of the stem taper is protected by the protector to avoid contamination or damage, as the trial head bore is arranged to accommodate the taper protector such that at least some or all of the articulating surface of the trial head and articulating surface of a corresponding prosthetic head will be coincidental. Otherwise, trialling from the prosthetic component may cause damage to the prosthetic component, either in the form of mechanical damage, or contamination of the interface surfaces. Hence, the surgeon can be surer that the articulating surface defined by the trial assembly will be very close to the articulating surface of the prosthetic assembly while reducing the chance of contamination or damage to the taper impairing the connection between the prosthetic stem and prosthetic head.

The trial bore can be non-tapered. For example, the trial bore can have the form of a circular cylinder.

The trialling system can further comprise a head prosthetic implant. The head prosthetic implant can have a prosthetic articulating surface and can include a tapered bore arranged to matingly receive the taper therein to form a prosthetic assembly of the stem prosthetic implant and the head prosthetic implant. The trial assembly can provides at least a portion, or all, of the trial articulating surface coincidental with the prosthetic articulating surface provided by the prosthetic assembly.

The taper can have a side wall and the protector can be in the form of a sleeve which covers all the side wall of the taper.

The taper can have a side wall and an end wall and the protector can be in the form of a cap which covers all the side wall of the taper and all or a part of the end wall of the taper.

The protector can have an outer surface arranged to engage within the trial bore. The inner surface can be resiliently deformable. The outer surface can be non-deformable or rigid.

The protector can have a single part or have a unitary construction. The protector can have regions of different hardness.

The protector can be made from the same type of material. Different regions or portions of the protector have different materials properties, such as hardness of deformability.

The protector can have two parts. A first part can be deformable. A second part can be non-deformable or rigid. The first part can be positioned within the second part.

The first part and second part can be made from the same type of material. The first part and the second part can have different materials properties, such as hardness or deformability

The first part can be made from a first material and the second part can be made from a second material different to the first material.

The first part can gave a tapering inner surface. The second part can have an outer surface which is a circular cylinder.

The inner surface can be shaped to provide an interference fit with the taper. For example, the inner surface can be a circular cylinder. The outer surface can be shaped to provide a taper fit with the trial bore. For example, the outer surface can be tapering.

The trialling system can further comprise an offset adjustment mechanism by which the position of the head trial implant along a longitudinal axis of the neck can be changed.

The offset adjustment mechanism can comprise a first formation and
a plurality of second formations. The plurality of second formations can be angularly disposed around the longitudinal axis. One of the first formation and the plurality of second formations can be provided on the neck and the other can be provided within the trial bore. Each of the second formations can matingly engage with the first formations to limit the depth of insertion of the neck into the trial bore by a different amount.

The first formation can be provided on the protector and the plurality of second formations can be provided within the trial bore of the head trial implant.

The first formation can be within the trial bore of the head trial implant and the plurality of second formations can be provided on the protector.

The first formation can be a male formation. For example, the first formation can comprise a lug. Each of the second formations can be female formations. Each of the second formations can comprise a slot. Each slot can have a different length.

The first formation can comprise a first pair of formations. The plurality of second formations can comprise a plurality of second pairs of formations

The head trial implant can include a plurality of markings. The plurality of markings can provide a scale. Each marking can correspond to a different offset associated with a respective one of the second pairs of formations.

The plurality of markings can be provided on a side part of the trial articulating surface. The plurality of markings can be angularly disposed about the longitudinal axis.

The neck can include a plurality of markings. The plurality of markings can provide a scale. Each marking can correspond to a different offset associated with a respective one of the second pairs of formations.

The plurality of markings can be disposed at different positions along the neck longitudinal axis.

Each second pair of formations can be arranged in diametric opposition around the longitudinal axis.

There can be at least three second pairs of formations, at least four, preferably at least five and more preferably at least six.

Embodiments of the invention will now be described in detail, by way of example only, and with reference to the accompanying drawings, in which:
Figures 1A to 1D show various views of a head part of a first example of a first trial system;
Figures 2A to 2D show various views of a neck part of the first trial system;
Figures 3A to 3C show perspective views of the first trial system in use;
Figures 4A to 4C shows various views of the first trial system adjusted to provide different amounts of offset;
Figures 5A to 5D show various views of a head part of a second example of the first trial system;
Figures 6A to 6D show various views of a neck part of the second example of the first trial system;
Figure 7 shows a side view of the first trial system illustrating marking on the head component;
Figure 8 shows a perspective view of the neck component of the first trial system illustrating neck marking;
Figure 9 shows a side view of the first trial system using the neck component of Figure 8;
Figure 10 shows a perspective view of parts of a first embodiment of a second trial system according to the invention;
Figure 11 shows a cross sectional view through parts of the second trial system of Figure 10;
Figure 12 shows a cross sectional view through the prosthetic components of the second trial system of Figure 10;
Figure 13 shows a cross sectional view through parts of an example of a third trial system;
Figure 14 shows a cross sectional view through parts of a second embodiment of the second trial system according to the invention;
Figure 15 shows a perspective view of parts of a third embodiment of the second trial system according to the invention;
Figure 16 shows a cross sectional view through parts of the third embodiment of the second trial system as shown in Figure 15;
Figure 17 shows a schematic perspective view of a taper protector and neck parts of a fourth embodiment of the second trial system according to the invention;
Figure 18 shows a schematic perspective view of a taper protector and neck parts of a fifth embodiment of the second trial system according to the invention;
Figure 19 shows a schematic perspective view of a taper protector and neck parts of a sixth embodiment of the second trial system according to the invention;
Figure 20 shows a schematic perspective view of an example of a protector part of the second trial system;
Figures 21A to 21D show various views of a head part, neck part and trial system according to a third example of the first trial system; and
Figures 22A to 22E show various views of a head part, neck part and trial system according to a fourth example of the first trial system.

Some embodiments of the invention will now be described by way of examples to provide an overall understanding of the principles of the structure, operation and use of the devices and methods disclosed herein. One or more examples of these embodiments are illustrated in the accompanying drawings. Those of ordinary skill in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Although the invention will be described below with reference to a hip implant, it will be appreciated that the invention can also be applied to other ball and socket type joint implants, such as a shoulder implant. In the following, "prosthesis" or "prosthetic" will be used to denote the actual implant finally placed by the surgeon to replace the patient's joint and "trial" will be used to denote for implants temporarily placed or used by the surgeon during the procedure for trialling purposes.

A first aspect of the invention aims to reduce the number of trial head components required to enable trialling of the different offsets and/or head sizes and/or taper sizes available. This is achieved by adapting the neck and trial head to allow the trial head to be attached at different depths to the neck and which correspond to the different offsets available of that head size. For example, for existing implant systems many different trial heads may be needed, for example up to twenty one to provide the different combinations of offset, taper size and head size needed to allow reliable trialling. However, this could be reduced to merely 7 different trial heads; one 28mm trial head; two 36mm trial heads; two 40mm trial heads; and two 44mm trial heads, with each trial head offering three different offsets. Fewer than seven different trial heads could be provided if more than three offsets are provided by the trial heads, particularly by the larger trial heads.

A second aspect of the invention aims to improve the reliability of trialling of ball and socket type joints, by allowing the surgeon to decide to use the prosthetic stem implant component for trialling rather than a trial stem component. A stem taper protector and specially adapted trial head component are used in which the trial head is configured to interact with the stem taper protector so that when assembled the prosthetic stem and trial head assembly has the same geometry as the prosthetic stem and a prosthetic head corresponding to the trial head. The stem taper protector allows the prosthetic stem to be used for trialling while protecting the stem taper from damage and/or being exposed to fluids from the surgical site which might otherwise decrease the reliability of the mating between the prosthesis stem taper and the prosthesis head component.

The first and second aspects of the invention can also be combined to provide offset adjustment and stem taper protection.

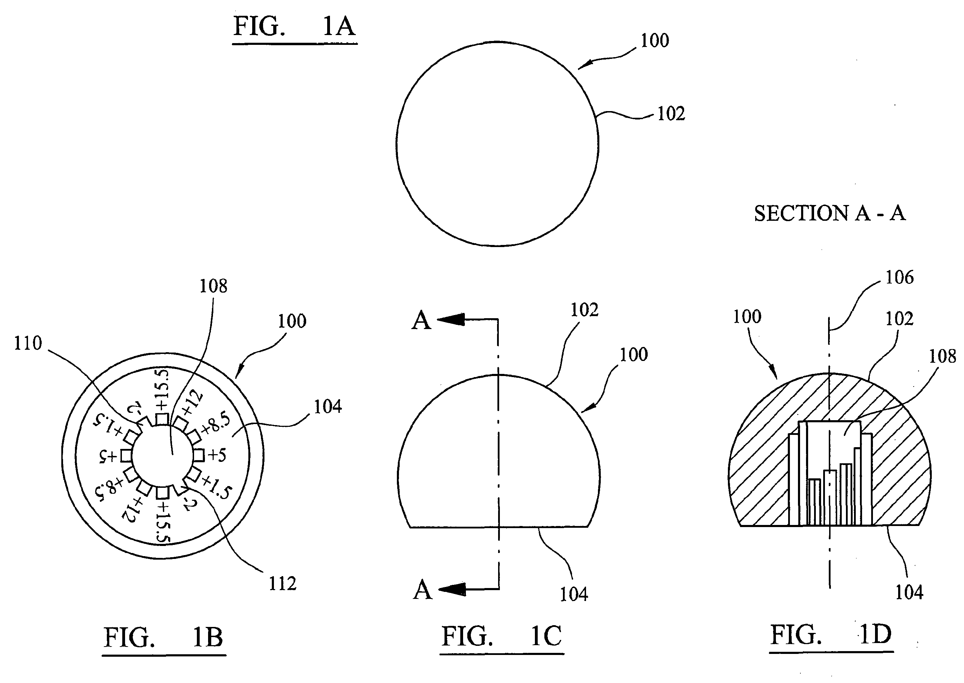

With reference to Figures 1A to 1D there are shown respective plan, bottom, side and cross sectional views of a first embodiment of a trial head 100 being a part of a trial implant system according to the first aspect of the invention. Trial head 100 has the form of a truncated sphere and has an articulating surface 102 extending over side and upper parts of the surface of a sphere. A lower surface 104 has a flat generally circular form. The trial head 100 has a longitudinal axis 106 passing through the centre of the lower circular surface 104 and through a pole in the upper part of the articulating surface 102 about which the articulating surface is generally rotationally symmetric.

As best illustrated in Figures 1B and cross section 1D (along line A-A of Figure 1C), a right circular cylindrical bore 108 is defined by an inner wall within the trial head and extends along the longitudinal axis. The inner wall of the trial head includes twelve slots, extending along the longitudinal axis 106 and arranged equi-angulalry around the longitudinal axis. The slots of each pair of the six opposed pairs of slots, e.g., slots 110 and 112, have the same length, and each pair of the six pairs of slots has a different length as best illustrated in Figure 1D. Each slot of a pair of slots is arranged diametrically opposite each other about the bore.

The trial head can be made of any suitable material, such as metals, alloys or plastics, particularly polymers. Preferably, the trial head is made from a material unlikely to damage the articulating surface of a socket component, in case a user trialled against a prosthetic implant, such as an acetabular cup. For example, the trial head can be made from a glass filed polyaryletherketone (PAEK) such as that provided under the name AvaSpire (a trade mark which may be registered in some countries), polyetheretherketone (PEEK) or polyphenylsulfone (PPSU) such as that provided under the name Radel (a trade mark which may be registered in some countries). The trial head 100 is provided in a plurality of different sizes, with each size corresponding to the size of a corresponding prosthetic head. The size of the head can be defined by the diameter of the spherical part. For example, the trial and prosthetic heads can be provided in the sizes 28mm, 36mm, 40mm and 44mm.

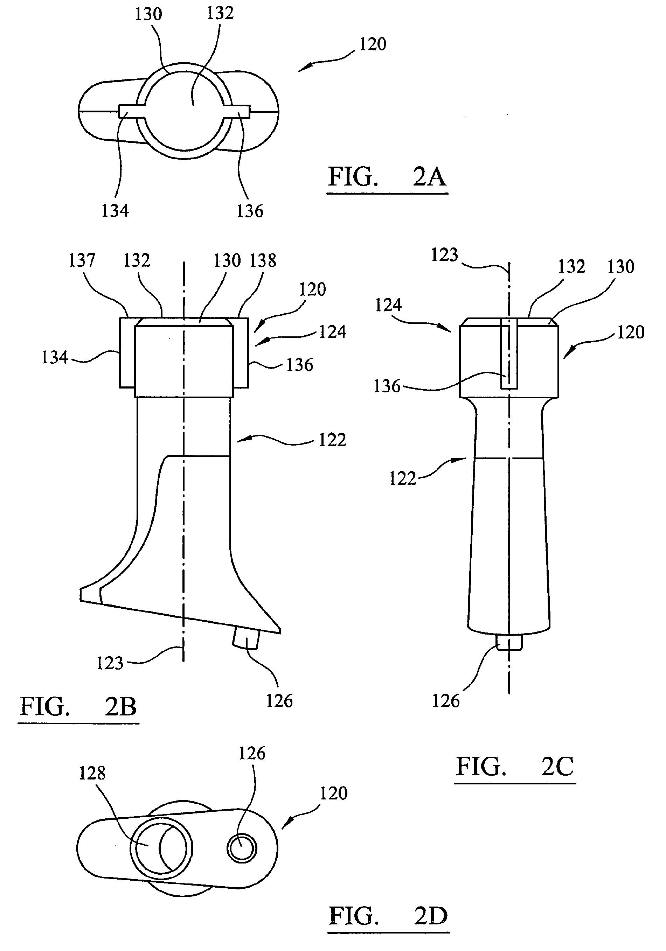

Figures 2A to 2D show respective plan, side, end and bottom views of a first stem component 120 being a part of the trial implant system.

The stem component includes a neck 122 having a free end 124 and a flared base 126. An underside of base 126 includes a first male attachment formation 126 and a second female attachment formation 128 for releasably attaching the stem component to mating features of a broach which has been used to prepare a cavity in a long bone to receive the stem prosthesis. The free end 124 of neck 122 has a generally right circular cylindrical form with a chamfered upper edge 130 extending around a top most circular surface 132. Neck 122 has a longitudinal axis 123 passing along its length and through the centre of circular surface 132. A first lug 134 and a second lug 136 extend from the side wall of the free end and provide a pair of lugs arranged diametrically opposite each other. The lugs have the same thickness and their thickness is similar to the width of each of the slots in the trial head so that the pair of lugs can be snuggly and slidingly received within each of the pairs of slots.

In an alternate example, the stem component 120 is provided as part of a stem trial implant rather than being a separate part which is attachable to a broach.

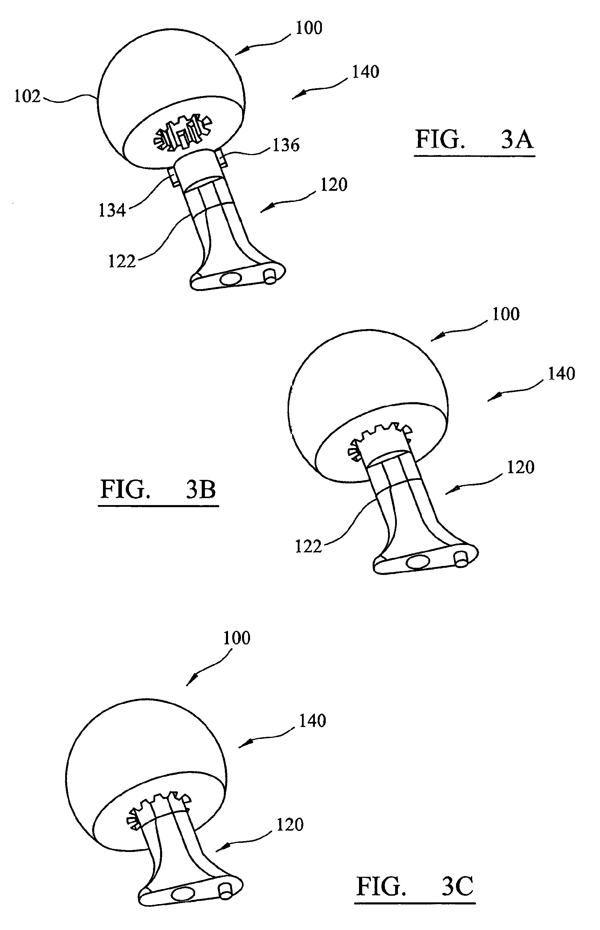

Figures 3A to 3C show respective perspective views of an assembly 140 of the trial head 100 and the stem component 120 during different stages of use. As illustrated in Figure 3A, the longitudinal axis 106 of bore 108 is aligned with the longitudinal axis 123 of neck 122 and with the trial head 100 rotated so as to align a one of the pairs of slots with the pair of lugs 134, 136 of the neck. The neck 120 is then introduced into bore 108 with the pair of lugs being slidingly received within respective slots of the pair of slots. The neck is progressed into the bore until upper ends 137, 138 of the lugs abut respective closed ends of the pair of slots to prevent further translation of the neck within the bore as illustrated in Figure 3C. This then controls the depth of insertion of the neck into the trial head and therefore the offset of the assembly of trial head and stem component, with the trial head at a particular angular position. The offset for the assembly is generally the difference between the extent of the implant assembly in a medial-lateral direction compared to a reference extent (a 'zero offset', although the implant assembly still has a finite extension in the medial lateral direction).

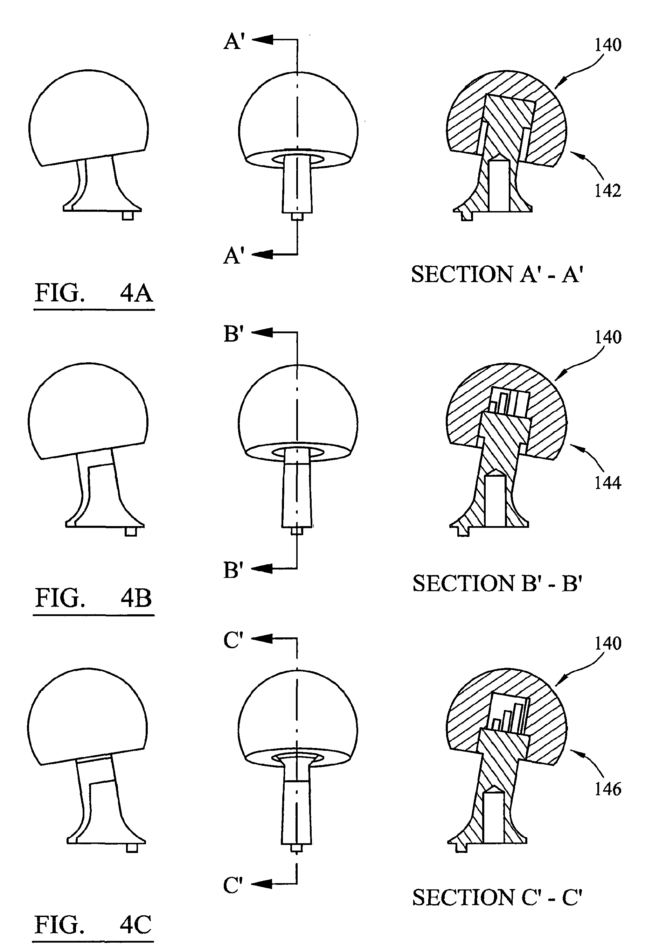

Figures 4A to 4C each show respective side, end and cross sectional views of assembly 140 with different amounts of offset. As each pair of slots has a different length, by changing the angle of rotation of the trial head about the longitudinal axis, a different one of the pairs of slots can be selected to be engaged by the pair of lugs to control the depth of insertion of neck 122 into bore 108. Figure 4A shows assembly 140 in a first maximal offset configuration 142 with the trial head in a first angular position in which the pair of lugs 134, 136 are received within a longest pair of slots of the six pairs of slots and corresponding to an increase in offset of 15.5mm. Figure 4B shows assembly 140 in an intermediate offset configuration 144 with the trial head in a second angular position in which the pair of lugs 134, 136 are received within a different pair of slots of the six pairs of slots and corresponding to an increase in offset of 5mm. Figure 4C shows assembly 140 in a minimal offset configuration 146 with the trial head in a third angular position in which the pair of lugs 134, 136 are received within a shortest pair of slots of the six pairs of slots and corresponding to a reduction in offset of 2mm.

As best illustrated in Figure 1B, the trial head includes six pairs of slots of different lengths and hence six different offsets (-2mm, +1.5mm, +5mm, +8.5mm, +12mm and +15.5mm) can be selected by changing the angular position of the trial head to one of six discrete angular positions. It will be appreciated, that in order to change the offset of the assembly, the trial head is translated away from the stem a sufficient distance to fully disengage the lugs from open ends of the slots. The trial head can then be rotated about its longitudinal axis to select a different offset and present a different pair of slots to the lugs. The trial head is then translated toward the stem component, with their respective longitudinal axes aligned, so as to engage the lugs with the different pair of slots and hence configure the assembly with an offset different to that of the previous configuration of the assembly.

As also illustrated in Figure 1B, the assembly can include markings showing the amount of offset associated with each of the different pairs of slots. In the first embodiment, the markings are provided on the under surface 104 of the trial head adjacent the open ends of the slots corresponding to the offsets. As shown in Figure 1B the markings can be numerical to indicated the amount of the offset and can also include a sign (positive or negative) to indicated whether the offset is an increase relative to an arbitrary zero (positive) or a decrease increase relative to the arbitrary zero (negative). Hence, for example, the pair of slots 110, 112 correspond to a reduction in offset of 2mm.

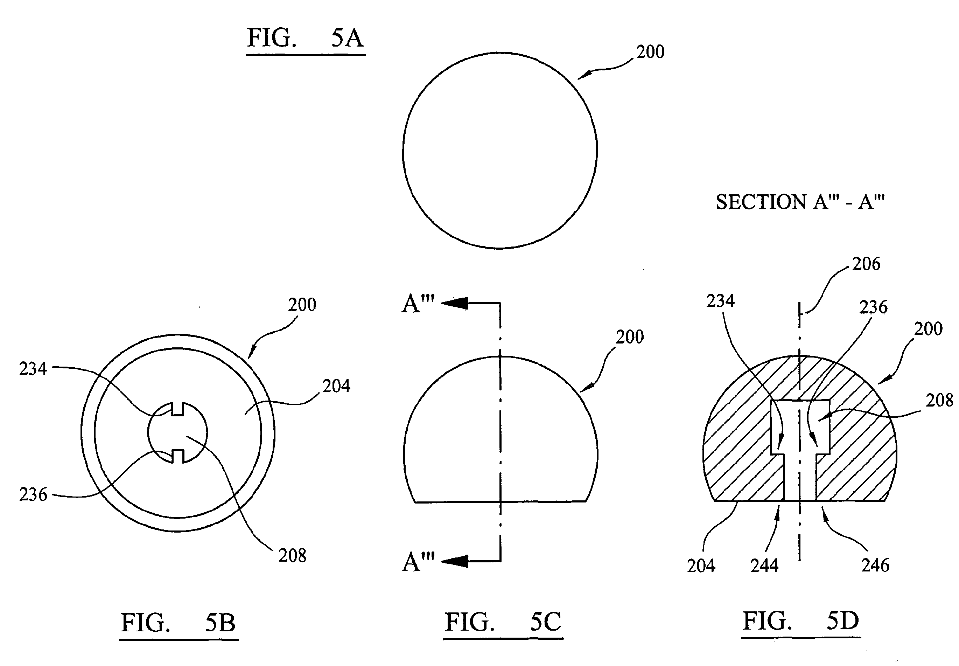

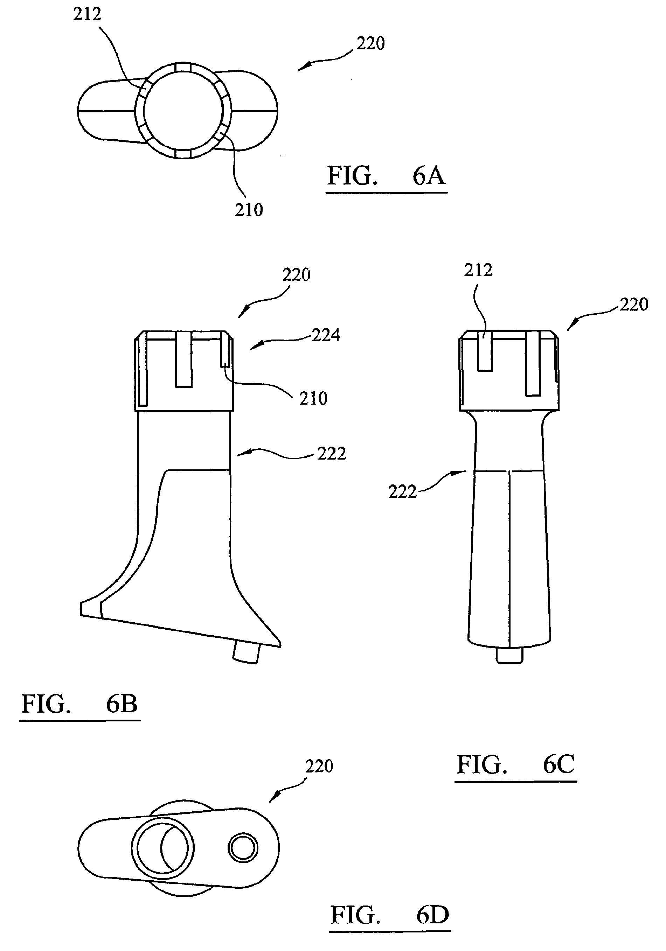

With reference to Figures 5A to 5D there are shown respective plan, bottom, side and cross sectional views of a second example of a trial head 200 being a part of a trial implant system. Also, Figures 6A to 6D show respective plan, side, end and bottom views of a second example of a stem component 220 being a part of the trial implant system. The second example of the trial head 200 and the stem component 220 are similar in construction to the first example described above. However, in the second example, the pair of lugs 234, 236 are provided on the trial head 200 and the plurality of pairs of slots, e.g. pair of slots 210, 212, are provided on the stem component 220.

As best illustrated in Figure 5D which is a cross sectional view along line A"' - A"' of Figure 5C, a pair of lugs 234, 236 extends from opposed sides of an inner side wall of the trial head 220 which defines an otherwise generally circular cylindrical bore 208 extending along a longitudinal axis 226 of the trial head 200. As illustrated in Figure 5D, the lugs extend from a lower edge 244, 246 flush with lower surface 204 to part way along bore 208. As illustrated in Figures 6A, 6B and 6C, the free end 224 bears 6 slots in total and which provide three pairs of slots, each pair having a different slot length thereby controlling the depth of insertion of neck 222 into bore 208 by abutment of lug lower ends 244, 246 with the closed ends of a one of the pairs of slots, e.g. slots 210, 212. The position of lower edges 244, 246 and/or the length of the lugs along the longitudinal axis 206 can be different. The position of the lower edges 244, 246 simply acts together with the position of the closed ends of the slots to control the depth of insertion of the neck 222 into bore 208.

It will be appreciated that the trial head 100, 200 has the same size as the corresponding prosthetic head that it is being used to trial for and hence presents the same articulating surface as the prosthetic head. Also the positions and lengths of the slots and lugs are arranged such that the trial assembly when configured to have a selected one of the offsets, e.g. +15mm, will have the same geometry and present the articulating surface at the same position and orientation as the prosthetic stem and head assembly with the corresponding offset, in this example +15mm.

In the first example, markings indicating the offset associated with a particular one of the pairs of slots are on the lower surface 104 of the trial head. However, in other examples other marking positions can be used, for example to increase the ease with which the markings can be seen by a surgeon while using the trial assembly.

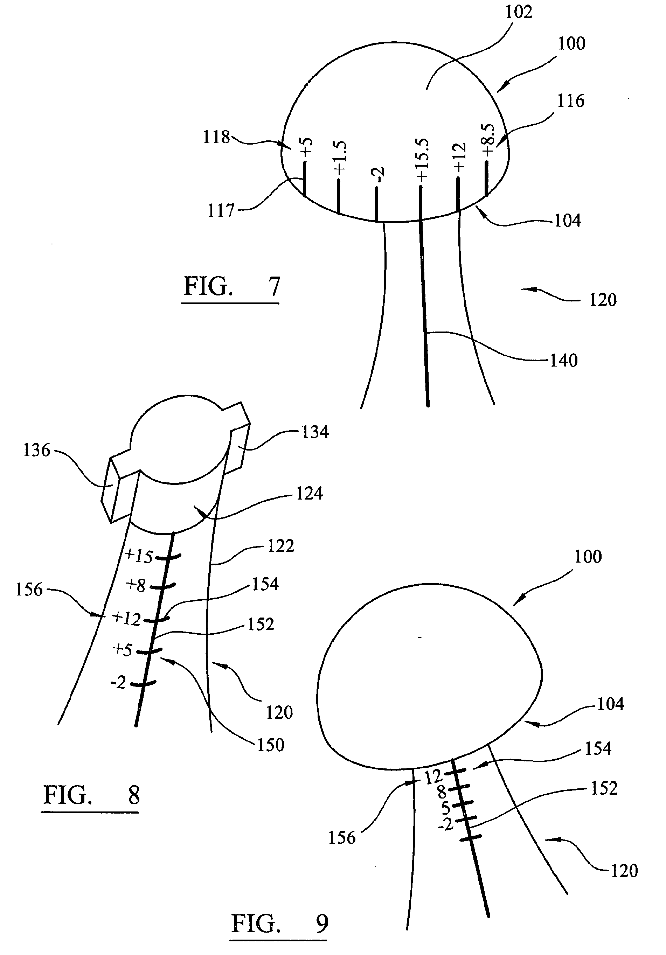

Figure 7 illustrates an alternative, or additional, marking scheme which can be used with the first example. The head 100 includes a plurality of markings 116 on a lateral or side portion of the articulating surface 102 and adjacent a junction with the lower surface 104. The plurality of markings 116 provide a scale from which an offset corresponding to a currently selected one of the pairs of slots can be read off. The markings 116 include a dash 117 and an indication of the sense (increase or decrease) and magnitude 118 of the offset placed to coincide with the position of the corresponding slot and arranged to abut the longitudinal axis 106 of the trial head. Equivalent markings are provided on the opposite side of the head and at diametrically opposed positions. The neck 120 includes a

single line marking 140 extending along the longitudinal axis 123 of the neck and aligned with a plane passing through the pair of lugs 134, 136. Hence, the marking 140 provides a reference or datum indicating the angular position of the lugs on the neck and which can be used to indicate the offset associated with a current configuration of the neck 120 and trial head 100 by reading off the one of the head markings 116 aligned with reference line 140, which is +15.5mm in the example illustrated in Figure 7.

Figures 8 and 9 illustrate a further alternative, or additional, marking scheme which can be used with the first or second example of the trial assembly. As illustrated in Figure
8, neck 124 of stem 120 includes a plurality of markings 150. The plurality of markings 150 provide a scale from which an offset corresponding to a currently selected one of the pairs of slots can be read off. The plurality of markings 150 include a linear marking 152 extending along the longitudinal axis 123 of the neck and a plurality of gradations, e.g. graduation 154, and an indication of the sense (i.e. increase or decrease in offset) and magnitude, e.g. 156, associated with each graduation. As illustrated in Figure 9, the graduations and markings are spaced along line 152 such that when the lugs 136, 134 are mated with and abutting the end of one of the pairs of slots, the position along the longitudinal axis of the lower face 104 of the trial head 100 is adjacent to the graduation 154 and indication 156 of the offset corresponding to the currently selected pair of slots, which in the illustrated example is +12. Hence any markings on the scale above are occluded by the trial head, while markings on the scale below are visible. Hence, the underside of the head and junction with the lateral part of the articulating surface act as the reference or datum by which to read from the scale the 'last' visible marking and which is adjacent the underside of the trial head.

Use of the trial implant system in the context of a hip arthroplasty procedure will now be described. The system can include a plurality of trial heads 100 of different sizes so as to accommodate the typical range of patient sizes. The system can also include trial heads of the same size but each having a different plurality of slots so that a full range of offsets is available for any particular head size. Although the trial implant system can include a plurality of trial heads, during use of the system not all of the trial heads may be used and indeed only a one of the trial heads may be used.

The femoral head is resected and a broach or sequence of broaches is used to create a cavity in the superior part of the femur. The broach includes a broach part and a releasably attachable handle. Once the cavity has been formed in the femur to the appropriate size and depth for the planned stem prosthesis, the broach part is left in the cavity and the broach handle is removed. The trial stem component 200 is then attached to the broach using attachment formations 128 and 126 which mate with interacting features on the broach. If the acetabulum is also being replaced, then the acetabulum is prepared and typically a trial cup is placed in the prepared acetabulum. The surgeon the selects a trial 100 head having the same size, i.e. diameter, as the trial cup and attaches the trial head to the stem component 100 at a first offset by engaging lugs 134, 136 with a first pair of slots. The surgeon may then reduce the joint to see whether the first offset and/or trial head size is appropriate. If the first offset is not appropriate for whatever reason, then the surgeon can remove the trial head, rotate the trial head and re-attach the trial head to select a different offset. The surgeon can then reduce the joint again to see if the newly selected offset is more appropriate. If the surgeon wants to try an offset not supported by the current trial head, then the surgeon can select another trial head with the same size but providing a different plurality of offsets. In that case, the surgeon removes and discards the first trial head, selects the new trial head, of the same size, and attaches the new trial head using the pair of slots corresponding to the offset to be trialled. Additionally, or alternatively, the surgeon may determine that the trial head size is not appropriate and may replace the initial trial cup with a further trial cup having a different size, for example larger, and then select a trial head suitable for use with the further trial cup. The surgeon may vary the combinations of offset and head/cup size until they are satisfied with the selected components and may then replace the trial components with prosthetic components having the determined size and offset.

Hence, the first aspect invention reduces the complexity and/or number of trial implants that are supplied while still allowing reliable trialling over a full range of typical patient sizes by providing a simple offset adjustment mechanism which can also allow the number of trial heads supplied into the operating room to be reduced.

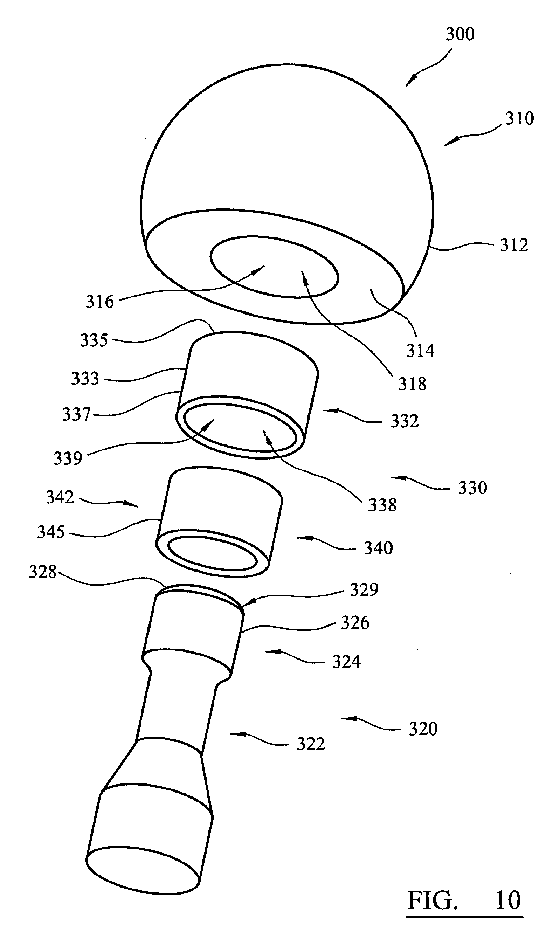

The present invention will now be described with reference to Figures 10 to 19, in particular. The second aspect of the invention also relates to trialling of ball and socket type joints. In particular the present invention provides the surgeon
with the option to decide to use the prosthetic stem component, rather than a trial stem component, for trialling with one or more trial head components.

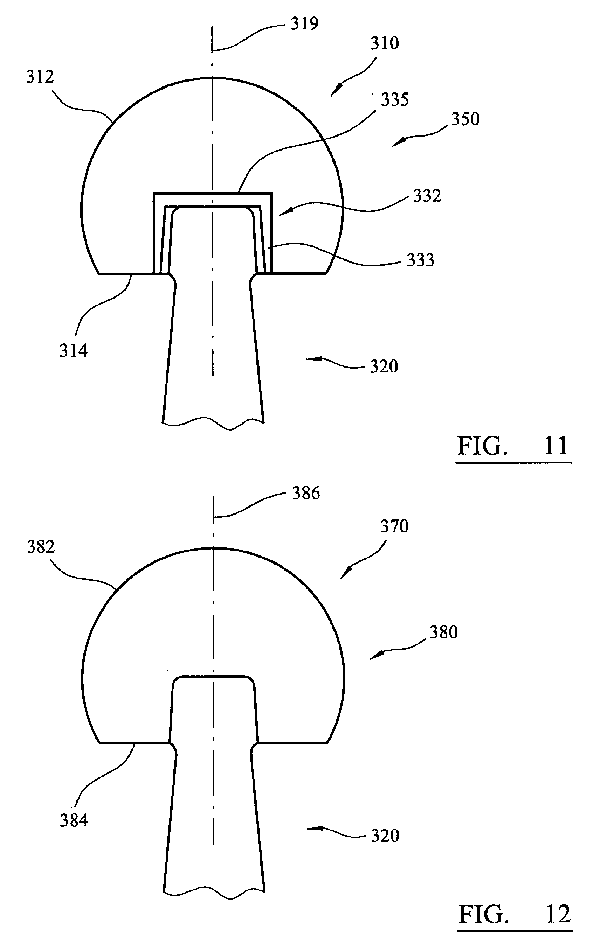

Figure 10 shows an exploded perspective view of a trial head part 310, prosthetic stem part 320 and stem protector part 330 of a trialling system 300 according to the present invention. Figure 11 shows a cross sectional side view through a trial assembly 350 of the trialling system. Figure 12 shows a cross sectional view through a prosthetic assembly 370 of the trialling system including the prosthetic stem part 320 and a prosthetic head 380.

The trial head 310 generally has the form of a truncated sphere and presents an articulating surface 312 extending over upper and side portions of the trial head. The trial head also includes a planar circular under surface 314. An inner wall 316 defines a circular cylindrical bore 318 which extends into the interior of the trial head along a longitudinal axis 319 which passes through the centre of under surface 314 and the pole at the top of articulating surface 312. The trial head has the same size as the prosthetic head as its spherical part has the same diameter as the spherical part of the prosthetic head 380. The trial head can be made from any suitable biocompatible material including plastics or polymers.

Figures 10, 11 and 12 show only the neck part of the prosthetic stem component 320 for the sake of clarity, although it will be appreciated that in practice the prosthetic stem also includes a main body part and shaft which extends into a cavity formed in the superior part of the femur. The neck part 322 of the prosthetic stem 320 includes a taper 324 toward a free end of the neck. The taper has a smooth side wall 326, and a circular end wall 328 and a chamfer 329 extending around the junction thereof. The finish of the taper side wall 326 is smooth so as to provide a strong interference fit with a corresponding tapered bore in the prosthetic head 380. The prosthetic stem can be made from a suitable biocompatible material such as a metal, an alloy or a plastic. For example, the stem can be made from forged or wrought titanium alloy.

The taper protector 330 has a first outer part 332 and a second inner part 340. Outer part 332 is made of a non-deformable rigid material. Outer part 332 has the form of a generally circular cylindrical cap with a side wall 333 and a circular end wall 335. An outer surface 337 of the side wall 333 is cylindrical and an inner surface 338 is slightly inclined and defines a tapered cavity 339 within the outer part 332. The outer part can be made from a suitable rigid or non-deformable biocompatible material such as a plastic or a metal or alloy. Various different engineering plastics can be used. Suitable plastics include silicone (especially with a high shore hardness), polyphenylsulfone (PPSU), such as that provided under the trade mark RADEL (which may be registered in some countries), Acetal and PEEK (Polyether Ether Ketone). Suitable metals and alloys include stainless steel, titanium and cobalt chromium alloys.

The inner part 340 is made from a deformable material and in particular can be made from a resiliently deformable material. The material of the inner part 340 is can be non-shedding and preferably will leave no-contamination or residue on the taper surfaces 326, 328. The inner part has the form of a generally tapered sleeve or tube, with open ends, and includes a tapering side wall 342. An outer surface 345 of the side wall 342 interfaces with the inner surface 338 of the outer part 332. An inner surface 346 of the side wall 342 interfaces with the outer surface 326 of the taper 324. The inner part 340 can be made from various deformable materials, such as silicone elastomer (with a low shore hardness), urethane, synthetic rubber, such as polyurethane rubber, and various foams. The inner part 340 has a lesser shore hardness than the outer part 330.

As best illustrated in Figure 11, the inner part 340 is attached within the outer part 330 and secured therein mechanically and/or by bonding, for example by an adhesive. The surfaces between the inner and outer part may be roughened to provide a better surface for an adhesive to key to. The inner part 340 and outer part 330 may be both made of the same type of material, e.g. silicone, but with different shore hardness, and they may be joined by a silicone adhesive so that the protector has a generally unitary construction.

As also well illustrated in Figure 11, the protector provides a deformable cavity or bore into which the taper 324 of the neck 322 is received and provides an interference fit by which the protector 330 is releasably secured to the taper 324. The protector then presents a non-deformable or rigid outer surface which can then be mated within the bore 318 of the trial head so as to protect the taper surfaces 326, 328 from being exposed to bodily fluids and/or potentially damaging contact with instrumentation or implants during trialling using the prosthetic stem 320.

With reference to Figure 12 there is shown a prosthetic assembly 370 comprising the prosthetic stem 320 and the prosthetic head 380. The prosthetic assembly 370 is assembled after trialling using the trial head 310 and prosthetic stem 320. The prosthetic head 380 has a similar construction to the trial head 310 in that it has a truncated spherical form including an articulating surface 382 and a flat under surface 384. A longitudinal axis 386 passes through the centre of the circular under surface 384 and through a pole toward the top of the articulating surface 382. An inner wall of the prosthetic head 380 defines a tapered cavity or bore which is dimensioned and shaped to provide a taper fit with the taper 324 of the prosthetic stem 320. The prosthetic head 370 can be made from any biocompatible material particularly including metals and alloys. Particularly suitable materials include ceramics, stainless steel, and Cobalt Chrome alloys.

The articulating surface 382 of the prosthetic head 370 is identical to the articulating surface 312 of the trial head 310. A difference between the trial head 310 and prosthetic head 380 is that the cavity 318 in the trial head is adapted to compensate for the presence of the protector 330 on the taper while ensuring that the position of the trial articulating surface 312 relative to the prosthetic stem component 320 is identical to the position of the prosthetic articulating surface 382 to the prosthetic stem 320 in the prosthetic assembly. That is, is Figure 12 and Figure 11 were overlaid, the respective articulating surface 312, 382 would be coincident over the entirety of the spherical surface portion of the trial head 310 and prosthetic head 380.

The structural differences between the trial cavity 318 and prosthetic cavity are that the prosthetic cavity is tapered whereas the trial bore 318 is in the form of a right circular cylinder. Also, the trial bore 318 has a greater lateral dimension and a greater depth so as to accommodate the side walls and end wall of the protector 330. The trial articulating surface 312 should include at least all of the prosthetic articulating surface 382. The trial articulating surface 312 can be greater than the prosthetic articulating surface 382 but should not be lesser so as to ensure that a full range of motion corresponding to that available with the prosthetic component can be trialled. The trial and prosthetic articulating surfaces are sufficiently similar such that the trial head does not behave differently to the prosthetic head.

Hence, as best illustrated by comparing Figures 11 and 12, the trial bore 318 is adapted to accommodate the protector 330 while ensuring that the articulating surface 312 of the trial assembly 350 is at the same position in space as the articulating surface 382 of the prosthetic assembly 370 over preferably the entirety of the common articulating surfaces. In particular, the trial cavity 318 has a greater depth and greater diameter than the prosthetic cavity. Also, the prosthetic cavity is tapered to ensure a good tapered interference fit between the taper 324 and prosthetic head 380 so that the prosthetic head 380 is securely attached to the prosthetic stem 320. The trial cavity 318 is cylindrical such that the trial head 310 can easily be removed from the prosthetic stem and replaced with a different trial head of a different size during the trialling procedure and without removing the protector 330 from the taper 324. The deformable part 340 helps to provide an interference fit between the protector 330 and taper which helps to prevent the protector being prematurely removed from the taper during trialling and before maturing the prosthetic head. Further, as the deformable part 340 is in the form of a sleeve, the non-deformable end wall 335 of the non-deformable part 332 is situated between the end face of the taper 328 and the end surface of the trial bore 318 which ensures accurate seating of the trial head 310 on the prosthetic stem 320 to ensure that the articulating surfaces of the trial and prosthetic heads are congruent. This is preferred compared to having a deformable part between the end surface of the taper 328 and the end face of the trial cavity which could lead to variation in position of the articulating surface of the trial head 310 when not seating on a rigid part.

Furthermore, the rigid outer walls 333 of the outer part 332 helps to retain the deformable part 340 within the protector 330 when the stem taper is initially introduced into the protector and enhances the interference fit by allowing the deformable part to be slightly compressed between the rigid taper surface 326 and the rigid side wall 333 of the outer part.

Hence, in use, the surgeon resects the proximal or superior part of the femur and creates a cavity in the resected femur using a broach or similar instrument. The prosthetic stem can then be placed in the cavity with the protector 330 in place and surrounding the side and end surfaces of the taper 324. The protector 330 can be provided pre-assembled to the prosthetic stem. In that case, the material of the protector is selected such that the protector does not degrade when sterilised or over time. The surgeon can then releasable attach trial heads of different sizes over the protector while being confident that the position of the trial articulating surface 312 will correspond to the position of the ultimately selected prosthetic head. After trialling, and when the surgeon has selected the appropriate head size, the protector 330 is removed from the taper 324 and the prosthetic head 370 is securely fastened to the prosthetic stem component 320 to form the prosthetic assembly 370 as illustrated in Figure 12. Owing to the use of the taper protector 330, the taper is not exposed to bodily fluids which may reduce or otherwise harm the effectiveness of the taper fit between the prosthetic head 370 and prosthetic stem 320. Additionally and / or alternatively, the taper protector 330 may help to avoid any scratches or other damage to the finish of the taper surfaces owing to instrumentation, implants or other equipment used by the surgeon during trialling. Therefore, the reliability of the attachment of the prosthetic head to the prosthetic stem can be improved and any issues associated with contamination or harm to the taper during trialling can be reduced or avoided.

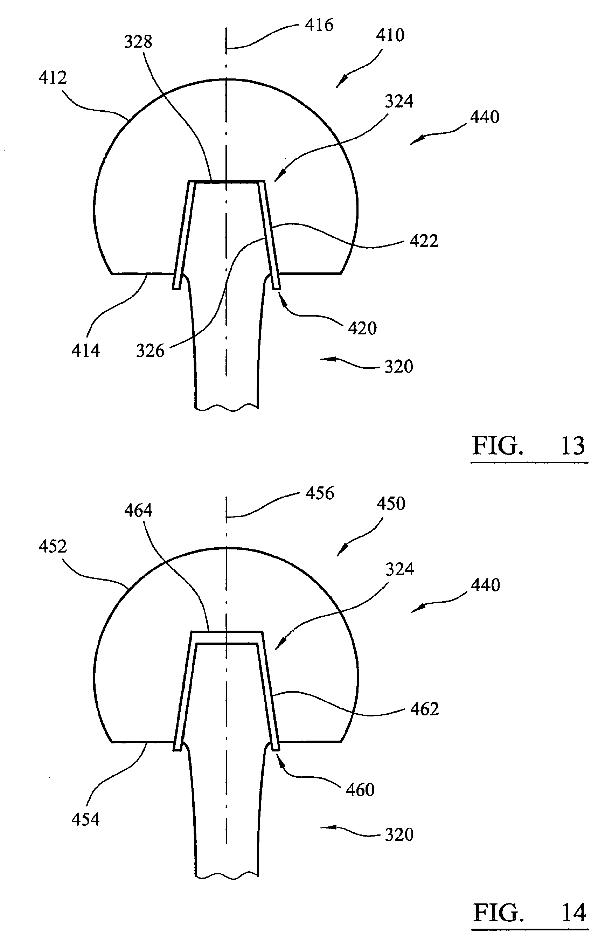

With reference to Figure 13 there is shown a further trial assembly 390 according to a further embodiment of the invention. The pile assembly 400 includes prosthetic stem 320 and trial head 410 having a spherical articulating surface 412 and a flat circular under surface 414. Trial head 410 is similar to the trial head 310 but has a tapered trial cavity extending into and along a longitudinal axis 416 of the trial head. Trial assembly 400 includes a protector 420 covering the side wall surface 326 of the taper 324. Taper protector 420 has the general form of a tapering sleeve or tube and comprises an inclined side wall 422. The taper protector 420 has open ends so that the inner end wall of the trial head bore can seat upon the upper end wall 328 of the taper. The taper protector 420 can have a number of constructions. It can be made from a resiliently deformable material or a rigid material or a combination thereof. The taper protector 420 can be made from the same type of material but engineered to have a deformable inner surface and a rigid outer surface. A deformable inner surface helps to provide interference fit between the protector 420 and the taper so as to help retain the protector on the taper during trialling. A rigid outer surface, and the shape of the tapered trial cavity can be engineered to provide a releasable attachment mechanism to allow different trial heads to be attached to and removed from the prosthetic stem 320 without pulling off the taper protector 420. In other embodiments, the sleeve 422 can have a circular cylindrical outer wall and a tapered inner wall similar to taper protector 330 but without the end wall 335. Although schematically illustrated in Figure 13 as being a single piece, in other embodiments, taper protector 420 may also have a two part construction including a rigid non-deformable outer part and a resiliently deformable inner part, again, similar to stem protector 330. Figure 14 shows a cross sectional view for a further embodiment of a trial assembly 440. Trial assembly 440 includes prosthetic stem 320 and trial head 350 similar to the previously described trial heads. Trial head 450 has a spherical articulating surface 452 and a flat circular lower surface 454 and a longitudinal axis 456 passing through the centre of circular under surface 454 and a pole of articulating surface 452. Trial head 450 includes inner surfaces defining a tapered trial bore within which a taper protector 460 and taper 324 of the prosthetic stem are received.

Taper protector 460 is similar to the taper protector 420 but has a generally cap-like construction and includes a tapering side wall 462 and a circular end wall 464. Taper protector 460 can be made from a non-deformable material, a deformable material or combinations thereof. Also, taper protector 460 can have a generally unity construction being made from a single part or may be made from two separate parts providing a deformable part and a non-deformable part, similar to taper protector 330. It is preferred if end wall 464 is made largely or entirely of a rigid material so as to provide a well-defined seating of the trial head 450 on the prosthetic stem 320 for the pile assembly 440 for the reasons discussed above.

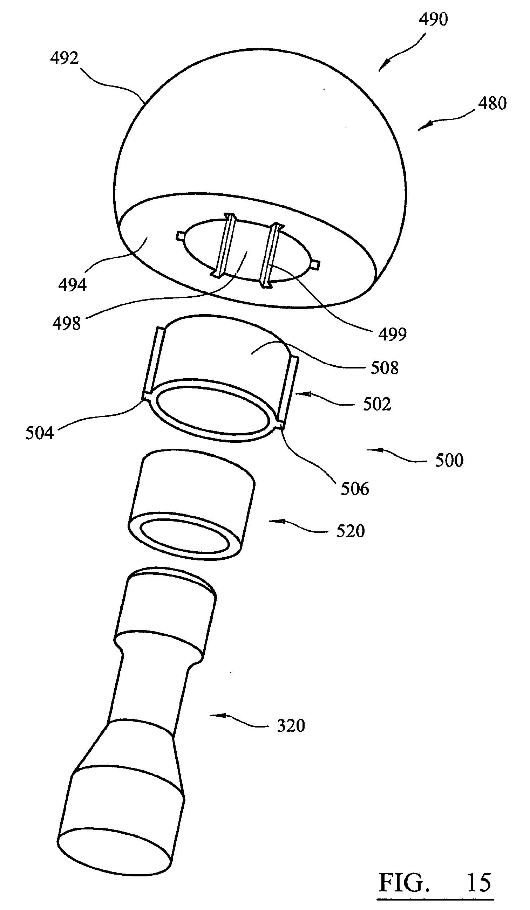

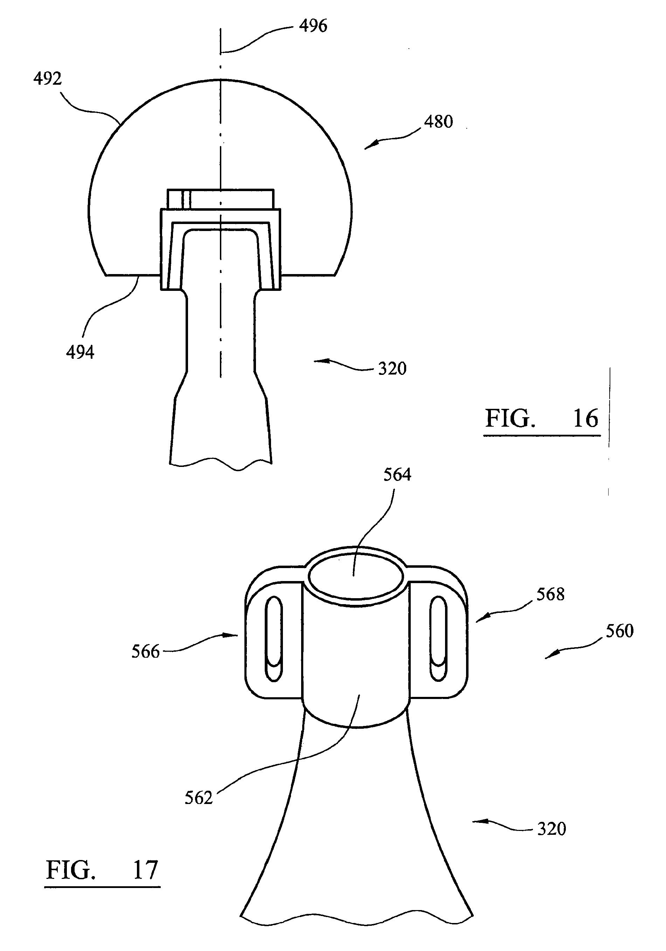

With reference to Figure 15, there is shown an exploded perspective view of a trial assembly 480 combining the first and second aspects of the invention. Figure 16 shows a cross sectional side view of the trial assembly 480. Trial assembly 480 includes a prosthetic stem 320, a trial head 490 and a taper protector 500. Trial head 490 has a spherical articulating surface 492, a circular under surface 494 and a longitudinal axis passing through the centre of circular under surface 494 and a pole of the articulating surface 492. Inner walls of trial head 490 define a trial cavity or bore 498 having a generally circular cylindrical shape. A plurality of slots, e.g. slot 499, are formed in an inner side wall of trial head 490 and define a plurality of pairs of slots of different length similar to those described for the first example.

Taper protector 500 has a similar construction to taper protector 330 and includes a non-deformable outer part 502 and an inner, tapered deformable part 520. Outer part 502 is made from a rigid material and has a cap like construction and also includes a pair or lugs or splines 504, 506 extending from a side wall 508 and diametrically opposed to each other.

Hence, as illustrated in Figure 16, the trial assembly 480 provides the combined benefits of the first and second aspects of the invention of allowing trialling from a prosthetic stem 320 or also providing offset adjustment and the benefits associated therewith described above.

Figure 17 shows a schematic perspective view of a further embodiment of a taper protector 560 mounted on the taper of a prosthetic stem 320. Taper protector 560 is similar to taper protector 500 but includes live spring elements to control the fit between the protector and the trial head. The live springs provide a snug fit between the protector and trial head, while also allowing for variability in the exact sizes owing to tolerances. As illustrated in Figure 17, the taper protector 560 has a generally cap-like form and includes a side wall 562 and a circular end wall 564. Side wall 562 has an outer surface presenting a circular cylindrical form and a tapered inner surface receiving an inner deformable part (not visible in Figure 17). A first 566 and a second 568 lug extends from diametrically opposed sides of the protector 560. Each lug 566, 568 includes an aperture passing therethrough to form the live spring elements.

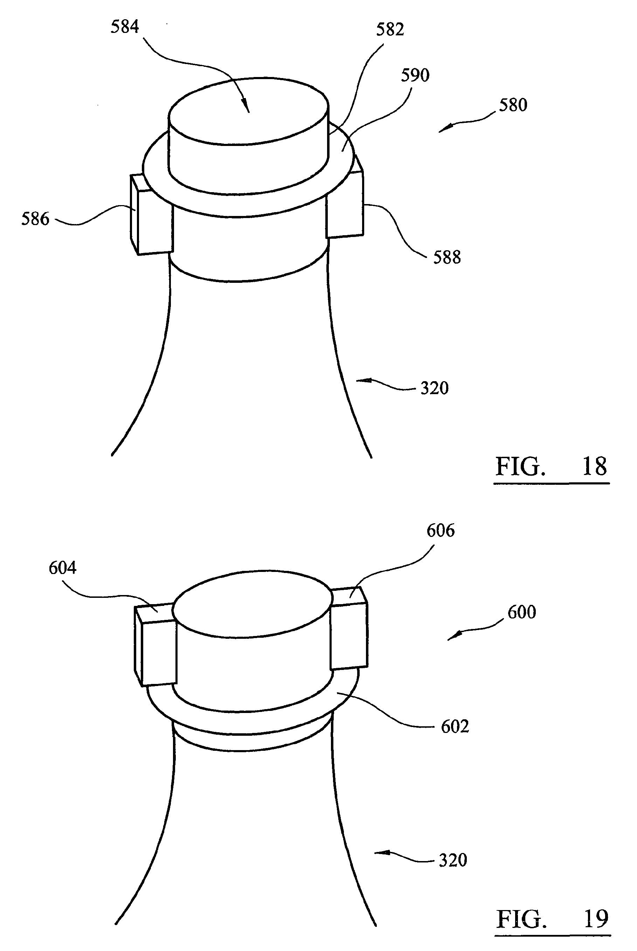

Figure 18 shows a schematic perspective view of a further embodiment of a stem protector 580 mounted on the taper of a prosthetic stem 320. Taper protector 580 has a similar construction to those described previously and has a generally cap-like form including a side wall 582 and a circular end wall 584. Taper protector 580 includes a pair of opposed lugs 586, 588 extending from a lower end of the taper protector 580 toward an intermediate portion and stopping short of the end wall 584. A circlip (sometimes also referred to as a c-clip) 590 is provided between the end wall 584 and an upper end of lugs 586, 588. Alternatively, an O-ring can be used instead of circlip 590. Similarly to the live springs, the circlip or O-ring provide a mechanism for controlling the fit between the protector and the trial head. Put another way, the O-ring or circlip help to control the interference fit between the parts.

Figure 19 shows a perspective view of a yet further embodiment of a taper protector 600 similar to taper protector 580. In the further embodiment, taper protector 600 includes a circlip 602 or O-ring extending around taper protector 600 but below lugs 604, 606. Otherwise, the construction of taper protector 600 is similar to that of taper protector 580 illustrated in Figures 18.

It will be appreciated that the features of protectors 560, 580 & 600 can also be used on the trial stem of the first aspect of the invention.

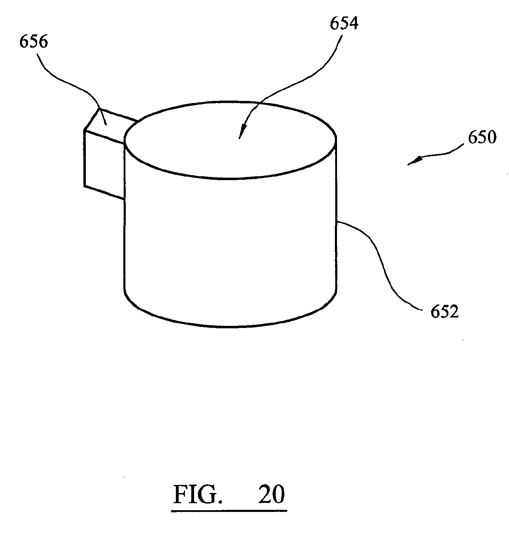

Figure 20 shows a schematic perspective view of a further example of a protector 650. Protector 650 has a generally circular cylindrical cap like construction and includes a side wall 652 and an end wall 654 and a single first formation 656 in the form of a lug extending outwardly form the side wall 656. It will be appreciated that protector 650 also includes a cavity for receiving a taper of a prosthetic stem in an interference fit manner similarly to that described above in connection with the various other embodiments of the protector. However, in this example, protector 650 includes only a single lug rather than a pair of lugs. Hence, the corresponding trial head includes a plurality of individual slots of different lengths, rather than pairs of slots of different lengths. It will be appreciated that a similar arrangement using a single male formation on a trial stem neck can also be used.

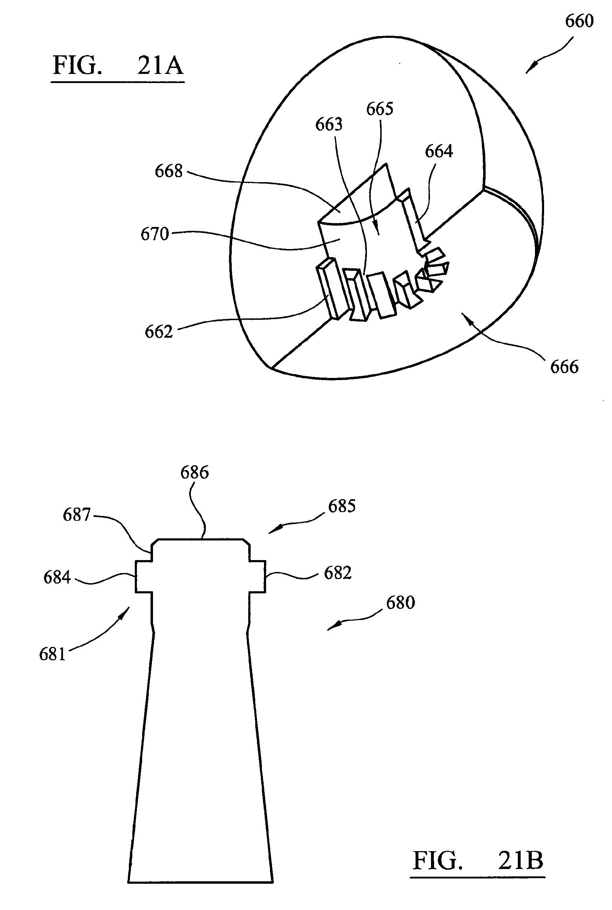

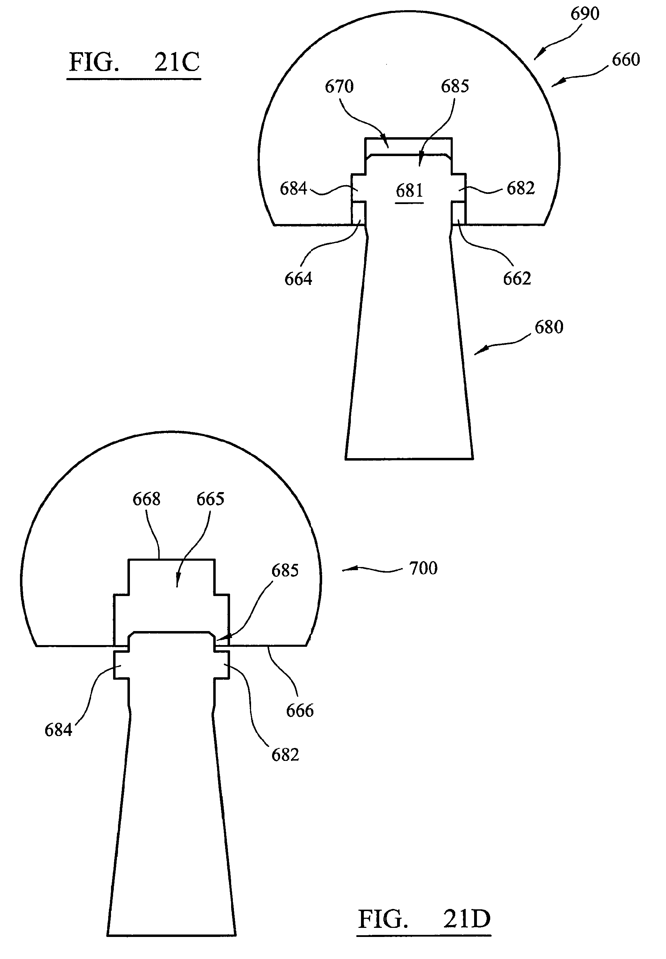

Hence, the example illustrated in Figure 20 uses only a single male formation interacting with a plurality of individual female formations, rather than pairs of male and female formations, in order to adjust offset. It will be appreciated that the alternate arrangement of the single male formation being in the cavity of the trial head and the individual female formations being on the protector or stem neck is also possible. Figures 21A to 21D show various views of a head part, neck part and trial system according to a third example of the invention. Figure 21A shows a perspective view cross sectional view of a trial head 660, Figure 21B shows a side elevation of a stem part 680, Figure 21C shows a side cross sectional view of the trial head and stem part in a fully engaged configuration or state 690 and Figure 21D shows a side cross sectional view of the trial head and stem part in a partially engaged configuration or state 700 in which the free end 685 of the stem 680 is still at least partially located or received within the central bore 665 of the head 660.

The third example is similar to the first example illustrated in Figures 1 to 4 and described above in that the trial head includes seven pairs of slots defined in an inner wall which defines a central, general circular bore 665. Each pair of slots comprises diametrically opposed slots, e.g. slots 662 664, each having the same length. However, each pair of slots has a different length compared to the other pairs of slots. All of the slots extend from an underside or under surface 666 of the trial head in a generally longitudinal direction parallel to the central axis of the trial head and terminate at a closed end which is part way into the central bore but not adjacent the end face 668 which closes the central bore 685. The longest pair of slots, slots 662, 664, extend approximately two thirds of the way into the bore such that approximately a third of the length of the side walls of the bore does not include any slots. This provides an end space 670 for receiving a free end of the stem as described in greater detail below.

The stem 680 is similar to the stem illustrated in Figures 2A to 2D, but the pair of lugs 682, 684 do not extend all the way to the top most surface 686 of the free end 685 of the cylindrical end portion 681 of the stem. Hence, there is a distal most portion of the stem at the free end 685 which does not bear any part of the lugs. The diameter of the cylindrical end portion 681 of the stem is slightly less than the inner diameter of the cylindrical bore 665 of the trial head so that the free end 685 can be slidingly received within the end space 670 and also within the remainder of the cylindrical bore 665. Figure 21C shows a side cross sectional view of the trial head 660 and stem part 680 in a fully engaged configuration or state 690. As can be seen, the lugs 684, 686 are received within a pair of slots 662, 664, and with the cylindrical end portion 681 of the stem located within the cylindrical bore of the trial head and in particular the free end portion 685 accommodated within the end space 670 of the cylindrical bore. The lugs and closed ends of the slots 662, 664 abut to control the depth of insertion of the stem into the trial head and therefore control the length or off set of the assembly. Figure 21C shows the minimal off set arrangement, i.e. using the longest pair of slots 662, 664, and hence the end space 670 has to be sufficiently long to accommodate the length of the free end part 685 of the stem.

In order to adjust the length of the assembly, the trail head is slid along the longitudinal axis, until the lugs 682, 684 have disengaged the slots and are clear of the under surface 666 of the trial head, as illustrated in Figure 21D. However, the free end portion 685 of the stem 685 is still located at least partially within the cylindrical bore 665 of the trial head and the trial head can be rotated about the free end portion 685 as the lands, e.g. land 663, between adjacent slots slide over the sidewall 687 of the free end portion 685. Hence, as illustrated in Figure 21D, the trial head 660 and stem part 680 are in a partially engaged configuration or state 700 in which they are not fully disengaged and in which the trial head can be rotated about the stem so as to allow a different pair of slots to be selected and engaged with the lugs 682, 684 to select a different length and hence off set for the trial assembly.

Allowing the length of the assembly to be changed without fully disengaging the trial head and stem makes it easier for the surgeon to adjust the length of the assembly *in situ* with the joint fully or partially reduced and without having to dislocate and then reduce the joint again. It is preferably to avoid repeatedly dislocating and/or reducing the joint during surgery so as to help avoid damage to the joint, such as to articulating surfaces and/ or tissue structures, such as tendons and ligaments.

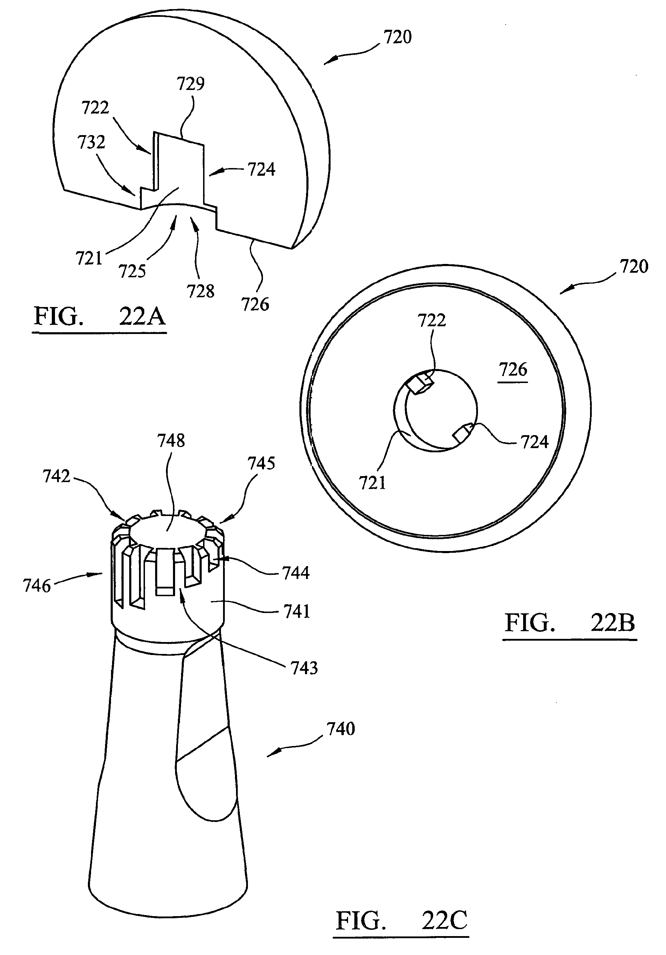

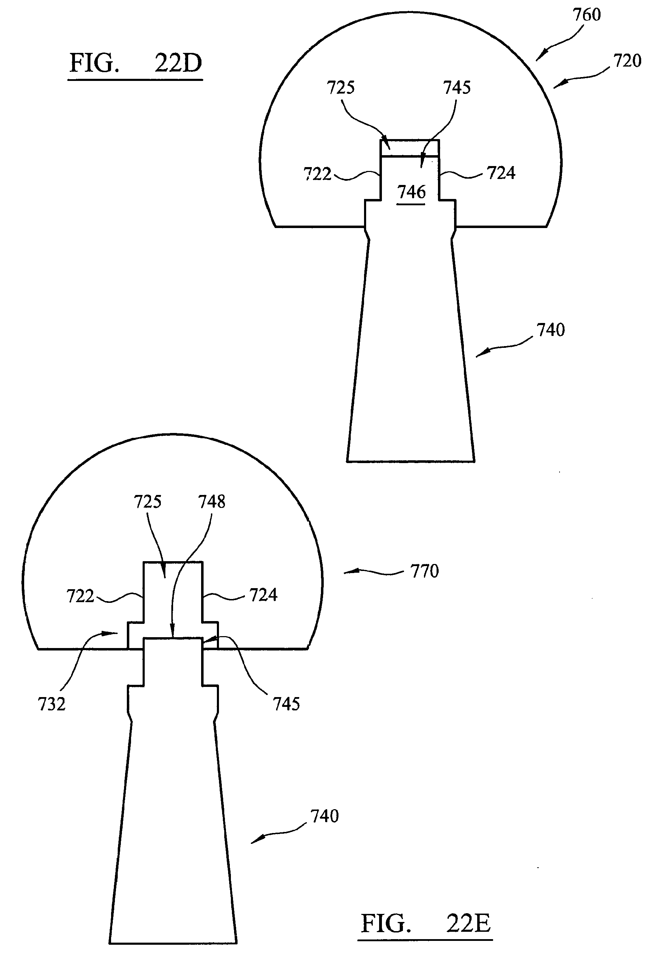

Figures 22A to 22E show various views of a head part, neck part and trial system according to a fourth example. Figure 22A shows a perspective cross sectional view of a trial head 720, Figure 22B shows a perspective view of an underside of the trial head 720, Figure 22C shows a perspective view of a stem part 740, Figure 22D shows a side cross sectional view of the trial head and stem part in a fully engaged configuration or state 760 and Figure 22E shows a side cross sectional view of the trial head and stem part in a partially engaged configuration or state 770 in which the free end 745 of the stem 740 is still at least partially located or received within the central bore 725 of the head 720.

With reference to Figure 22A and 22B, the fourth example is similar to the second example illustrated in Figures 5 and 6 and described above in that the trial head 720 includes a pair of lugs 722, 724 extending inwardly from an inner wall 721 which defines a central, general circular bore 725. The trail head 720 is similar to that shown in Figure 5 except the lugs do not extend all the way down to an under surface 726 of the trail head. Rather, the lugs 722, 724 terminate before the under surface 726 at an open end 728 of the bore and not adjacent the end face 726 which defines the mouth to the central bore 725. The lugs extend approximately two thirds to three quarters of the way along the bore from the closed end 729 toward the open end such that approximately a third or a quarter of the length of the side walls of the bore does not include any part of the lugs. This provides an open end space 732 for receiving a free end of the stem as described in greater detail below.

With reference to Figure 22C, the stem 740 is generally similar to the stem illustrated in Figures 6A to 6D, and includes six pairs of slots, e.g. pair of diametrically opposed slots 742, 744, defined in an outer wall 746 which defines a generally circular cylindrical end portion 746 having a free end portion 745. Each pair of slots comprises diametrically opposed slots, e.g. slots 742 744, each having the same length. However, each pair of slots has a different length compared to the other pairs of slots. All of the slots extend from a top most face or surface 748 of the cylindrical end portion 746 of the stem in a generally longitudinal direction parallel to the central axis of the stem and terminate at a closed end which is part way along the end portion 746.

The diameter of the cylindrical end portion 746 of the stem is slightly less than the inner diameter of the cylindrical bore 725 of the trial head so that the free end 745 can be slidingly received within the open end space 732 and also within the remainder of the cylindrical bore 725.

Figure 22D shows a side cross sectional view of the trial head 720 and stem part 740 in a fully engaged configuration or state 760. As can be seen, the lugs 722, 724 are received within a pair of slots and with the cylindrical end portion 746 of the stem located within the cylindrical bore 725. The lugs and closed ends of the slots abut to control the depth of insertion of the stem into the trial head and therefore control the offset of the assembly. Figure 22D shows the minimal off set arrangement, i.e. using the longest pair of slots, and hence the fee end 745 of the stem is received within the bore but separated from the closed end face 729.

In order to adjust the length of the assembly, the trail head is slid along the longitudinal axis, until the lugs 722, 724 have disengaged the slots and are clear of the top end surface 748 of the trial head, as illustrated in Figure 22E. However, the free end portion 745 of the stem 740 is still located at least partially within the cylindrical bore 725 of the trial head, within the open end space 732 and the trial head can be rotated about the free end portion 745 as the lands, e.g. land 743, between adjacent slots slide over the inner wall 721 of the bore. Hence, as illustrated in Figure 22E, the trial head 720 and stem part 740 are in a partially engaged configuration or state 770 in which they are not fully disengaged and in which the trial head can be rotated about the stem so as to allow a different pair of slots to be selected and engaged by the lugs 722, 724 to select a different length and hence off set for the trial assembly.

Hence, this example also allows the length of the assembly to be changed without fully disengaging the trial head 720 and stem 770. The surgeon can therefore adjust the length of the assembly *in situ* with the joint fully or partially reduced and without having to dislocate and then reduce the joint again, thereby reducing the chance of damage to the joint.

It will be appreciated that the same function can also be realised by modifying the slots rather than the lugs. For example the position of the open ends of the slots can be modified so that they terminate before the end most surface of the neck, similarly to the lugs shown in Figure 21B. Various modifications to the positions and/or lengths of the lugs and or slots can be used to allow an end part of the neck to remain in the bore during adjustment of the trial assembly and hence not fully separating the trial assembly during its adjustment.

It will be appreciated that the features of the different embodiments may be modified and/or used in different combinations to those of the embodiments specifically described above.

## Claims

1. A trialling system (300), comprising:
a stem prosthetic implant (320), having a neck (322) bearing a taper (324) having an end face (328);
a protector (330, 460, 500) covering at least a part of the taper and being removable from the taper, wherein the protector has a side wall (342, 462) and an end wall (335, 464), and wherein the end wall is rigid; and
a head trial implant (310, 450, 490) having a trial articulating surface (312, 452, 492) and including a trial bore (318) arranged to matingly receive the taper covered by the protector therein to form a trial assembly (350, 440, 480) of the stem prosthetic implant, wherein the trial bore has an end face and is wider than the taper and longer than the taper and the head trial implant (310) is releasably attachable to the protector (330),
wherein an inner surface (346) of the side wall (342, 462) is deformable and engages the taper, **characterised in that** the inner surface (346) of the side wall (342, 462) is less hard than the end wall (355, 464), and wherein the end wall (335,464) is configured to be situated between the end face of the taper and the end face of the trial bore to ensure accurate seating of the trial head on the prosthetic stem when the head trial implant (310) is assembled on the protector (330) on the taper (324) to form the trial assembly (350, 440, 480).

2. The trialling system (300) as claimed in claim 1, and further comprising:
a head prosthetic implant (380) having a prosthetic articulating surface and including a tapered bore arranged to matingly receive the taper therein to form a prosthetic assembly (370) of the stem prosthetic implant and the head prosthetic implant and in which the trial assembly provides at least a portion of the trial articulating surface coincidental with the prosthetic articulating surface provided by the prosthetic assembly (370).

3. The trialling system as claimed in claim 1, wherein the taper (324) has a further side wall and the protector (330, 500) includes a deformable sleeve (340, 520) having the inner surface of the side wall and which covers all the further side wall of the taper.

4. The trialling system as claimed in claim 1, wherein the taper has a further side wall and the protector (330, 460, 500) is in the form of a cap which covers all the further side wall of the taper and all the end face of the taper.

5. The trialling system as claimed in claim 1, wherein the protector (330, 500) has a yet further side wall (333, 508) and an outer surface of the yet further side wall and the end wall (335) are arranged to engage within the trial bore (318) and wherein the outer surface is rigid.

6. The trialling system as claimed in claim 1, wherein the protector (460) has a single part having regions of different hardness.

7. The trialling system as claimed in claim 6, wherein the protector (460) is made from the same type of material.

8. The trialling system as claimed in claim 1, wherein the protector (330, 500) has a first part (340, 520) which is deformable and a second part (332, 502) which is rigid.

9. The trialling system as claimed in claim 8, wherein the first part (340, 520) is made from a first material and the second part (332, 502) is made from a second material different to the first material.

10. The trialling system as claimed in claim 8, wherein the first part (340, 520) is tapered and the second part (332, 502) has an outer surface (337) which is a circular cylinder.

11. The trialling system as claimed in claim 1, wherein the inner surface (346) is a circular cylinder which provides an interference fit with the taper (324) and the outer surface is tapered and provides a taper fit with the trial bore.

12. The trialling system as claimed in claim 8 or 9, wherein the first part (340, 520) is silicone elastomer, urethane, synthetic rubber, polyurethane rubber or foam.

13. The trialling system as claimed in claim 1, and further comprising:
an offset adjustment mechanism by which the position of the head trial implant (490) along a longitudinal axis (496) of the neck can be changed.

14. The trialling system as claimed in claim 13, wherein the offset adjustment mechanism comprises:
a first pair of formations (504, 506, 566, 568, 586, 588, 604, 606); and
a plurality of second pairs of formations (499) angularly disposed around the longitudinal axis (496), wherein one of the first pair of formations and the plurality of second pairs of formations is provided on the neck and the other is provided within the trial bore and wherein each of the second pairs of formations can matingly engage with the first pair of formations to limit the depth of insertion of the neck into the trial bore by a different amount.

15. The trialling system as claimed in claim 14, wherein the first pair of formations (504, 506, 566, 568, 586, 588, 604, 606) comprises a pair of male formations, the plurality of second pairs of formations comprises a plurality of pairs of female formations (499), the pair of male formations are provided on the protector (500, 560580, 600), the plurality of pairs of female formations (499) are provided within the trial bore (498).

## Patentansprüche

1. Erprobungssystem (300), umfassend:
ein Schaftprothesenimplantat (320), das einen Hals (322) mit einer Verjüngung (324) aufweist, die eine Stirnseite (328) aufweist;
einen Protektor (330, 460, 500), der zumindest einen Teil der Verjüngung abdeckt und von der Verjüngung entfernbar ist, wobei der Protektor eine Seitenwand (342, 462) und eine Endwand (335, 464) aufweist, und wobei die Endwand starr ist; und
ein Kopfprobeimplantat (310, 450, 490), das eine Probegelenkfläche (312, 452, 492) aufweist und eine Probebohrung (318) enthält, die angeordnet ist, die durch den Protektor abgedeckte Verjüngung passend in sich aufzunehmen, um eine Probeanordnung (350, 440, 480) des Schaftprothesenimplantats zu bilden, wobei die Probebohrung eine Stirnseite aufweist und breiter als die Verjüngung und länger als die Verjüngung ist, und das Kopfprobeimplantat (310) lösbar an dem Protektor (330) anbringbar ist,
wobei eine Innenfläche (346) der Seitenwand (342, 462) verformbar ist und in die Verjüngung eingreift,
**dadurch gekennzeichnet, dass** die Innenfläche (346) der Seitenwand (342, 462) weniger hart ist als die Endwand (355, 464), und wobei die Endwand (335, 464) konfiguriert ist, sich zwischen der Stirnseite der Verjüngung und der Stirnseite der Probebohrung zu befinden, um einen akkuraten Sitz des Probekopfs an bzw. auf dem Prothesenschaft sicherzustellen, wenn das Kopfprobeimplantat (310) an bzw. auf dem Protektor (330) an bzw. auf der Verjüngung (324) montiert ist, um die Probeanordnung (350, 440, 480) zu bilden.

2. Erprobungssystem (300) nach Anspruch 1, und ferner umfassend:
ein Kopfprothesenimplantat (380), das eine Prothesengelenkfläche aufweist und eine verjüngte Bohrung enthält, die angeordnet ist, die Verjüngung passend in sich aufzunehmen, um eine Prothesenanordnung (370) des Schaftprothesenimplantats und des Kopfprothesenimplantats zu bilden, und bei dem die Probeanordnung zumindest einen Abschnitt der Probegelenkfläche übereinstimmend mit der Prothesengelenkfläche bereitstellt, die von der Prothesenanordnung (370) bereitgestellt wird.

3. Erprobungssystem nach Anspruch 1, wobei die Verjüngung (324) eine weitere Seitenwand aufweist und der Protektor (330, 500) eine verformbare Hülse (340, 520) enthält, welche die Innenfläche der Seitenwand aufweist und welche die gesamte weitere Seitenwand der Verjüngung abdeckt.

4. Erprobungssystem nach Anspruch 1, wobei die Verjüngung eine weitere Seitenwand aufweist und der Protektor (330, 460, 500) in der Form einer Kappe ist, welche die gesamte weitere Seitenwand der Verjüngung und die gesamte Stirnseite der Verjüngung abdeckt.

5. Erprobungssystem nach Anspruch 1, wobei der Protektor (330, 500) noch eine weitere Seitenwand (333, 508) aufweist und eine Außenfläche der noch weiteren Seitenwand und die Endwand (335) angeordnet sind, in der Probebohrung (318) in Eingriff zu kommen, und wobei die Außenfläche starr ist.

6. Erprobungssystem nach Anspruch 1, wobei der Protektor (460) einen einzigen bzw. einzelnen Teil mit Regionen unterschiedlicher Härte aufweist.

7. Erprobungssystem nach Anspruch 6, wobei der Protektor (460) aus demselben Materialtyp besteht.

8. Erprobungssystem nach Anspruch 1, wobei der Protektor (330, 500) einen ersten Teil (340, 520), der verformbar ist, und einen zweiten Teil (332, 502) aufweist, der starr ist.

9. Erprobungssystem nach Anspruch 8, wobei der erste Teil (340, 520) aus einem ersten Material besteht und der zweite Teil (332, 502) aus einem zweiten Material besteht, das sich von dem ersten Material unterscheidet.

10. Erprobungssystem nach Anspruch 8, wobei der erste Teil (340, 520) verjüngt ist und der zweite Teil (332, 502) eine Außenfläche (337) aufweist, die ein Kreiszylinder ist.

11. Erprobungssystem nach Anspruch 1, wobei die Innenfläche (346) ein Kreiszylinder ist, der eine Presspassung bzw. Interferenzpassung mit der Verjüngung (324) bereitstellt, und die Außenfläche verjüngt ist und eine Kegelpassung mit der Probebohrung bereitstellt.

12. Erprobungssystem nach Anspruch 8 oder 9, wobei der erste Teil (340, 520) Silikonelastomer, Urethan, Synthesekautschuk, Polyurethankautschuk oder Schaumstoff ist.

13. Erprobungssystem nach Anspruch 1, und ferner umfassend:
einen Versatzeinstellmechanismus, durch den die Position des Kopfprobeimplantats (490) entlang einer Längsachse (496) des Halses verändert werden kann.

14. Erprobungssystem nach Anspruch 13, wobei der Versatzeinstellmechanismus umfasst:
ein erstes Paar Formationen (504, 506, 566, 568, 586, 588, 604, 606); und
eine Mehrzahl zweiter Paare Formationen (499), die winkelig um die Längsachse (496) angeordnet sind, wobei eines des ersten Paars Formationen und der Mehrzahl zweiter Paare Formationen an bzw. auf dem Hals bereitgestellt ist und das andere innerhalb der Probebohrung bereitgestellt ist, und wobei jedes der zweiten Paare Formationen passend in das erste Paar Formationen eingreifen kann, um die Einsetztiefe des Halses in die Probebohrung um einen unterschiedlichen Betrag zu begrenzen.

15. Erprobungssystem nach Anspruch 14, wobei das erste Paar Formationen (504, 506, 566, 568, 586, 588, 604, 606) ein Paar aufzunehmende Formationen umfasst, die Mehrzahl zweiter Paare Formationen eine Mehrzahl von Paaren aufnehmender Formationen (499) umfasst, das Paar aufzunehmender Formationen an bzw. auf dem Protektor (500, 560580, 600) bereitgestellt ist, die Mehrzahl von Paaren aufnehmender Formationen (499) innerhalb der Probebohrung (498) bereitgestellt sind.

## Revendications

1. Système d'essai (300), comprenant :
un implant prothétique à tige (320), ayant un col (322) portant un cône (324) ayant une face d'extrémité (328) ;
un dispositif de protection (330, 460, 500) couvrant au moins une partie du cône et pouvant être retiré du cône, dans lequel le dispositif de protection comprend une paroi latérale (342, 462) et une paroi d'extrémité (335, 464), et dans lequel la paroi d'extrémité est rigide ; et
un implant d'essai à tête (310, 450, 490) ayant une surface d'articulation d'essai (312, 452, 492) et incluant un alésage d'essai (318) agencé pour recevoir par accouplement le cône couvert par le dispositif de protection pour former un ensemble d'essai (350, 440, 480) de l'implant prothétique à tige, dans lequel l'alésage d'essai a une face d'extrémité et est plus large que le cône et plus long que le cône et l'implant d'essai à tête (310) peut être fixé de manière détachable au dispositif de protection (330),
dans lequel une surface interne (346) de la paroi latérale (342, 462) est déformable et se met en prise avec le cône, **caractérisé en ce que** la surface interne (346) de la paroi latérale (342, 462) est moins dure que la paroi d'extrémité (355, 464), et dans lequel la paroi d'extrémité (335, 464) est configurée pour être située entre la face d'extrémité du cône et la face d'extrémité de l'alésage d'essai pour garantir une assise précise de la tête d'essai sur la tige prothétique lorsque l'implant d'essai à tête (310) est assemblé sur le dispositif de protection (330) sur le cône (324) pour former l'ensemble d'essai (350, 440, 480).

2. Système d'essai (300) selon la revendication 1, et comprenant en outre :
un implant prothétique à tête (380) ayant une surface d'articulation prothétique et incluant un alésage conique agencé pour recevoir par accouplement le cône pour former un ensemble prothétique (370) de l'implant prothétique à tige et de l'implant prothétique à tête et dans lequel l'ensemble d'essai fournit au moins une partie de la surface d'articulation d'essai coïncidente avec la surface d'articulation prothétique présentée par l'ensemble prothétique (370).

3. Système d'essai selon la revendication 1, dans lequel le cône (324) possède une autre paroi latérale et le dispositif de protection (330, 500) inclut un manchon déformable (340, 520) présentant la même surface interne que la paroi latérale et qui recouvre l'ensemble de l'autre paroi latérale du cône.

4. Système d'essai selon la revendication 1, dans lequel le cône possède une autre paroi latérale et le dispositif de protection (330, 460, 500) se présente sous la forme d'un capuchon qui couvre l'ensemble de l'autre paroi latérale du cône et l'ensemble de la face d'extrémité du cône.

5. Système d'essai selon la revendication 1, dans lequel le dispositif de protection (330, 500) possède encore une autre paroi latérale (333, 508) et une surface externe de l'encore autre paroi latérale et de la paroi d'extrémité (335) sont agencées pour se mettre en prise à l'intérieur de l'alésage d'essai (318) et dans lequel la surface externe est rigide.

6. Système d'essai selon la revendication 1, dans lequel le dispositif de protection (460) possède une partie unique ayant des régions de dureté différente.

7. Système d'essai selon la revendication 6, dans lequel le dispositif de protection (460) est constitué du même type de matériau.

8. Système d'essai selon la revendication 1, dans lequel le dispositif de protection (330, 500) possède une première partie (340, 520) qui est déformable et une seconde partie (332, 502) qui est rigide.

9. Système d'essai selon la revendication 8, dans lequel la première partie (340, 520) est constituée d'un premier matériau et la seconde partie (332, 502) est constituée d'un second matériau différent du premier matériau.

10. Système d'essai selon la revendication 8, dans lequel la première partie (340, 520) est conique et la seconde partie (332, 502) a une surface externe (337) qui est un cylindre circulaire.

11. Système d'essai selon la revendication 1, dans lequel la surface interne (346) est un cylindre circulaire qui permet un ajustement serré avec le cône (324) et la surface externe est conique et permet un ajustement conique avec l'alésage d'essai.

12. Système d'essai selon la revendication 8 ou 9, dans lequel la première partie (340, 520) est de l'élastomère de silicone, de l'uréthane, du caoutchouc synthétique, du caoutchouc ou de la mousse de polyuréthane.

13. Système d'essai selon la revendication 1, et comprenant en outre :
un mécanisme de réglage de décalage au moyen duquel la position de l'implant d'essai à tête (490) le long d'un axe longitudinal (496) du col peut être changée.

14. Système d'essai selon la revendication 13, dans lequel le mécanisme de réglage de décalage comprend :
une première paire de formations (504, 506, 566, 568, 586, 588, 604, 606) ; et
une pluralité de secondes paires de formations (499) disposées de manière angulaire autour de l'axe longitudinal (496), dans lequel l'une de la première paire de formations et de la pluralité de secondes paires de formations est placée sur le col et l'autre est placée à l'intérieur de l'alésage d'essai, et dans lequel chacune des secondes paires de formations peut se mettre en prise par accouplement avec la première paire de formations pour limiter la profondeur d'insertion du col dans l'alésage d'essai dans une mesure différente.

15. Système d'essai selon la revendication 14, dans lequel la première paire de formations (504, 506, 566, 568, 586, 588, 604, 606) comprend une paire de formations mâles, la pluralité de secondes paires de formations comprend une pluralité de paires de formations femelles (499), la paire de formations mâles est placée sur le dispositif de protection (500, 560, 580, 600), la pluralité de paires de formations femelles (499) sont placées à l'intérieur de l'alésage d'essai (498).
